Europäisches Patentamt

European Patent Office

Office européen des brevets

(11) Publication number: **0 377 762**
**A1**

# EUROPEAN PATENT APPLICATION

(21) Application number: **89100308.9**

(22) Date of filing: **10.01.89**

(51) Int. Cl.⁵: **C12N 1/00**

(43) Date of publication of application:
**18.07.90 Bulletin 90/29**

(84) Designated Contracting States:
**AT BE CH DE FR GB IT LI NL SE**

(71) Applicant: **KUBOTA TEKKO KABUSHIKI
KAISHA**
**No. 2-47, Shikitsu-higashi 1-chome
Naniwa-ku
Osaka-shi Osaka-fu(JP)**

(72) Inventor: **Hiyamuta, Shota**
**29-1, Kohchi-daigaku shukusha 200
Mononobe-otsu
Nangoku-shi Kohchi-ken(JP)**
Inventor: **Shirakawa, Noboru**
**29-7, Tezukayama 1-chome
Nara-shi Nara-ken(JP)**
Inventor: **Itoh, Hajime**
**27-603, Asahigaokacho 1-chome
Suita-shi Osaka-fu(JP)**
Inventor: **Uenaka, Susumu**
**11-9, Tsukunocho 3-cho
Sakai-shi Osaka-fu(JP)**

(74) Representative: **Vossius & Partner**
**Siebertstrasse 4 P.O. Box 86 07 67
D-8000 München 86(DE)**

(54) A continuous process for culturing microorganisms.

(57) A continuous process for growing microorganisms in a circulatory system is disclosed wherein a CSL solution is used which is neutralized by an alkaline solution introduced immediately upstream of where the CSL solution is supplied and/or into the circulatory system, and wherein the pH values of the neutralized solution and the culture nutrient liquid in the circulatory system are constantly detected and controlled in order to permit the constant addition of an adequate amount of the alkaline solution.

# A CONTINUOUS PROCESS FOR CULTURING MICROORGANISMS

The present invention relates to a continuous process for culturing microorganisms and more particularly, to a process adapted for growing bacteria particularly acid bacteria on a mass-production basis, wherein the acid bacteria include lactic acid bacteria and acetic acid bacteria.

To grow microorganisms, a batch culturing process is well known and widely used, in which viable bacteria are placed in a culture medium in a vessel. The batch process is designed to grow the bacteria in a closed space. The major disadvantage of the batch process is that the mass-production of bacteria is difficult because of the limited capacity of the vessel. Take an example for lactic acid bacteria: if they grow to be as much as 4 to 5g per 1 liter of culture medium ($2 \times 10^9$ CFU/m$\ell$ in terms of the number) they no longer grow because of the excessively increased concentration of lactic acid and density of bacteria. If the bacteria is to be mass-produced on the batch basis, several batches are required, depending upon the quantity to be produced, and each batch needs a series of steps: that is, sterilizing, placing, discharging and cleaning. This is time- and labor-consuming.

In order to overcome the disadvantage of the known batch process, it is proposed to omit some of the steps mentioned above, and continue to culture in the same vessel by keeping the concentrations of the growing bacteria and the resulting product, and the pH value in the vessel as constant as possible.

However, as far as the same culture bed is used, the growing bacteria are likely to be contaminated with harmful germs. The proposed process is therefore only adapted for culturing yeast or any other species which are not liable to harmful germs. As is generally known, lactic acid bacteria and other acid bacteria are easily spoiled by harmful germs, and the proposed process is not applicable to the culture of acid bacteria.

To produce microorganisms on a mass-production basis, there is another proposal which is characterized by the use of a hollow cylindrical filter made of ceramic or any other porous material. The filter is designed to grow microorganisms in a dense state, from which a filtrate is obtained.

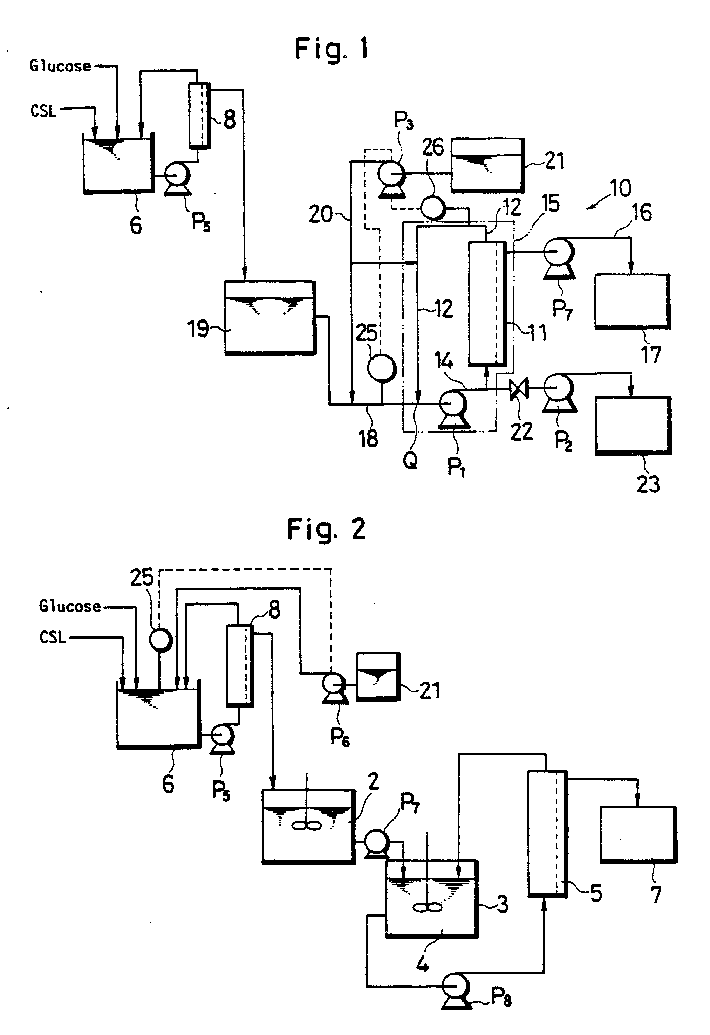

There is a further proposal, which employs the batch process and the filter in combination, which will be more particularly described by reference to Fig. 2:

A sterilized culture bed 2 is placed in a nutrient liquid 4 held in a vessel 3, wherein the pH value of the bed 2 is adjusted to 6.8 or around. There is provided a filter 5, and the nutrient liquid 4 is circulated between the filter 5 and the vessel 3. A filtrate is collected from the filter 5, and introduced into a reservoir 7. A product is separated from the filtrate. The reference numerals 6, 8, 21 and 25 denote a pH adjusting vessel, a second filter, an alkaline solution and a pH controller, respectively. There are provided pumps $P_5$ to $P_8$.

According to this combination process, microorganisms can be mass-produced to some extent. Nevertheless, the problem is that the culture bed 2 is quickly saturated with the growing microorganism. As a result, a second batch, a third batch and so on, depending upon the quantity to be produced, must be prepared. As described above, the pH value of the culture bed 2 must be neutralized before it is introduced in the vessel 3. This neutralization of culture bed 2 prior to entering the vessel 3 requires a complicated procedure. For example, when a corn steeping liquid bed (having a concentration of 5%) (hereinafter called "CSL") is acidified to 6.8 pH, a precipitate containing sodium phytic acid is produced, thereby increasing the amount of suspended solids from 1.8g/$\ell$ up to 6.2g/$\ell$. As a result, an undesirable film is formed to block up the meshes of the filter. The precipitate also chokes the pipes and valves in the equipment. In addition, the neutralization of the culture bed allows harmful germs to grow, which spoils the growing microorganism. Tests show that lactic acid bacteria are particularly susceptible to damage by the harmful germs.

Acid bacteria require a sufficient quantity of a nutrient to promote the growth. The CSL serves as a nutrient but it is inherently an acid substance. To employ it as a culture nutrient bed, it is required to increase its pH value to 6.8 or around. The neutralization produces a precipitate, which likewise causes a choking trouble in the piping and valves. As a result, it is required to remove the precipitate after the pH value of the CSL solution is adjusted. The precipitate contains a valuable nutrient for culturing microorganisms, and if the precipiate is discarded, the nutrient nevertheless will be wasted.

The present invention is directed toward a process for culturing microorganisms which solve the problems discussed above with respect to the known batch process. An object of the present invention is to provide a continuous process for culturing microorganisms in a CSL culture bed, thereby promoting the growth of bacteria.

Another object of the present invention is to provide a continuous process for mass-producing microorganisms under a safe condition from harmful germs.

According to the present invention, there is provided a continuous process for growing microorganisms in a circulatory system, the process comprising supplying a CSL solution, extracting some of the CSL solution containing viable bacteria, adding an alkaline solution to the CSL solution immediately before the CSL solution is introduced into the circulatory system so that the CSL solution is neutralized, and detecting the pH values of the neutralized solution and the nutrient in the circulatory system so that an adequate amount of the alkaline solution is constantly added.

Fig. 1 is a flowchart showing a culturing system for carrying out the process according to the present invention;

Fig. 2 is a flowchart showing a culture system for use in carrying out a known batch process;

Fig. 3 is a graph showing a relationship between the number of bacteria and the optical densities of culture beds;

Fig. 4 is a graph showing a relationship between the optical densities and the ratios of extraction to supply;

Fig. 5 is a graph showing a relationship between the number of bacteria and the ratios of extraction to supply; and

Fig. 6 is a flowchart showing another example of the culturing system.

As shown in Fig. 1, the system 10 for carrying out the process of the present invention includes a circulatory system 15 which is provided with a hollow cylindrical filter 11, a first conduit 12, a pump $P_1$ and a second conduit 14. The filter 11 functions as a culturing vessel, in which viable acid bacteria (hereinafter represented by lactic acid bacteria) grow in a dense state. The culture nutrient liquid containing the growing bacteria is circulated through the circulatory system 15.

The culture bed containing a product (i.e. lactic acid) produced in the filter 11 is filtered through the meshes of the filter 11, and the filtrate is drafted and stored in a reservoir 17, wherein the amount of draft is adjusted by a pump $P_7$ so as to maintain the constant conditions in the circulatory system 15 for growing the lactic acid bacteria.

A CSL solution 19 is supplied into the circulatory system 15 through the conduit 18, wherein the CSL solution 19 has a pH value of 4 or around. The CSL solution 19 is intended as a sterile culture bed (nutrient liquid). To neutralize the CSL solution 19, an alkaline solution 21 (e.g. 10N-NaOH) is added thereto at a place immediately upstreams of a point Q until the pH value is increased to 6.8 or around. Before the solution 19 reaches the point Q, its acidity is strong enough to prevent harmful germs from growing, thereby protecting the solution 19 against a possible contamination by harmful germs. It is required to control the amount of the CSL solution so as to maintain an optimum condition for growing the lactic acid bacteria by taking into consideration the amount of the filtrate drafted by the pump $P_7$.

Owing to the neutralization of the CSL solution at the point Q, that is, immediately before its entering the circulatory system 15, the piping and valves in the equipment are free from a choking trouble with a possible precipitate. The neutralization of CSL solution produces a precipitate containing a sodium phytic acid, but at the point Q where it encounters with a low pH culture medium passing through the conduit 12, its pH value is again reduced so that the sodium phytic acid is decomposed into sodium and phytic acid. The separated phytic acid again dissolves in the CSL solution 19. The precipitate containing the rest of sodium phytic acid is introduced into the filter 11 as a nutrient, thereby making most use of the CSL solution as a nutrient.

In general when the bacteria in a closed vessel grow in a dense state, an excessively increased density of microorganism spontaneously stops a further growth. Under the present invention a surplus of the culture medium containing the growing bacteria is continuously or intermittently extracted from the circulatory system 15 through a valve 22 by means of a second pump $P_2$ and the extracted solution is stored in a second reservoir 23. In this way the present invention allows a given quantity of acid solution 19 to be supplied on one hand, and on the other hand, a portion of the growing bacteria in the filter 11 is extracted so as to keep the concentration of bacteria in the filter 11 in an optimum condition to grow.

It is important to adjust the pH value of the CSL solution to an appropriate value. To facilitate the adjustment of pH value, the pH value controller 25 is provided at a place downstreams of where the alkaline solution 21 is added to the CSL solution 19, which is upstreams of the point Q. The pH controller 25 detects the pH value of the CSL solution neutralized by the alkaline solution 21, and, in response to the detected pH value, controls the supply of the alkaline solution 21 by the pump $P_3$. It is also important to keep the pH value in the filter 11 at 4.5 to 5.5. However if the filter 11 receives the CSL solution having an inappropriate pH value, the pH value therein will likewise become inappropriate. To avoid this problem, a pH detector 26 is also disposed on the conduit 12 so as to detect the pH value of the culture bed from the filter 11. In this way the rate of the alkaline solution 21. When the pH value of the filtrate from the filter 11 is in an optimum range, it is understood that the pH of the sterile zone starting from the conduit 18 is also in an optimum range. If the pH values of the filtrate and the sterile zone are constantly related to, the pH detector 25 can be omitted, and the pH detector 26 on the conduit 12 suffices. In general, the pH values upstream of the point Q tends to fluctuate in a larger range, and to secure an accurate detection it is possible to provide the conduit 26 alone with the pH detector 26. It is also possible to provide a pH detector in the conduit 14 at the inlet port of the filter 11 so as to detect the pH value of the culture bed supplied to the filter 11. When several pH detectors are provided, they are preferably interconnected to control each other.

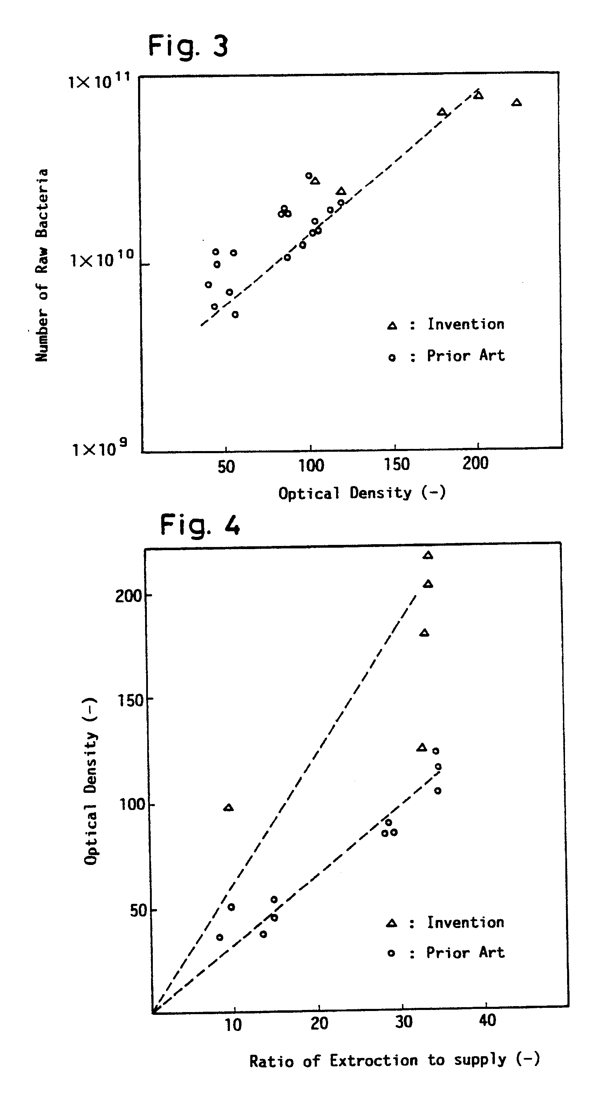

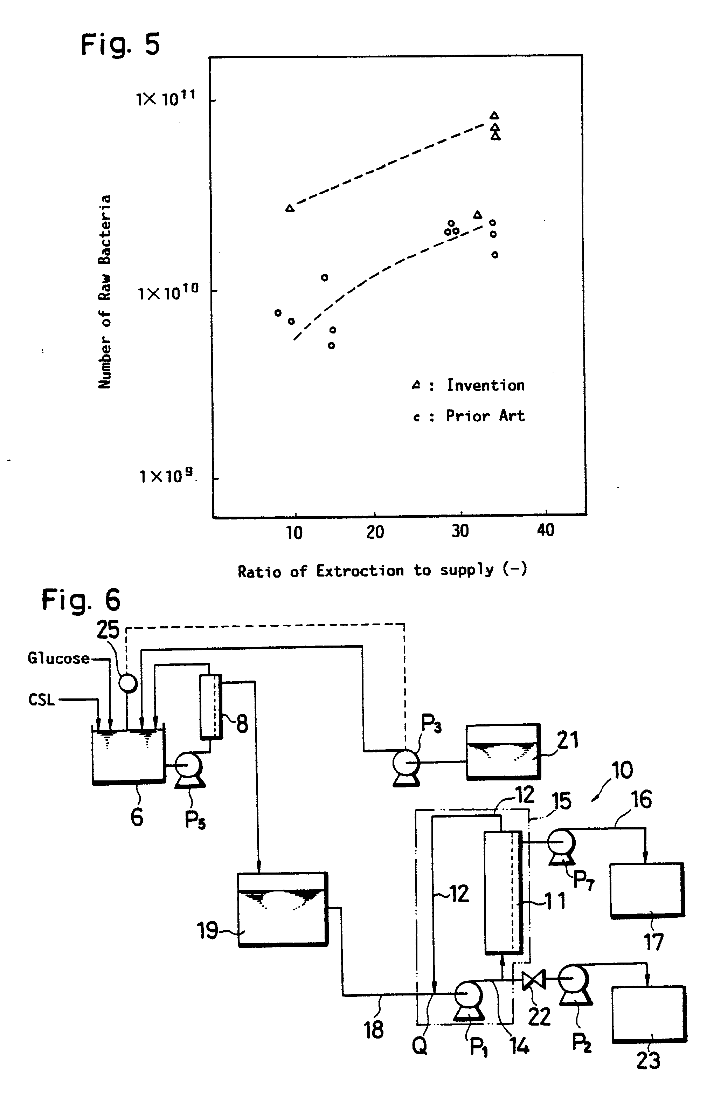

The number of microorganism growing in the equipment 10 was examined and represented for each ratio of extraction to supply in Figs. 3 to 5. The data is shown in contrast to those obtained from a known equipment. The phrase "ratio of extraction to supply" means a ratio of the extraction of that culture bed containing growing bacteria from the circulatory system 15 to the supply of the sterile culture bed thereto for a unit period of time. The number was obtained by inspecting viable bacteria in the reservoir 23 in terms of CFU/ml. In depicting the graph of Fig. 3 a given range was previously determined in which the relationship exists between the number of viable bacteria and the optical density (Fig. 3), and the optical density was obtained for each rate of extraction (Fig. 4), wherein the reference optical density was 660nm in wavelength, and 18mm pass, employing a UV-120-01 type (manufactured by Shimazh Mfg. Co., Ltd.). Based upon the results shown in Figs. 3 and 4, the graph of Fig. 5 was depicted to show a relationship between the number of viable bacteria and the ratio of extraction to supply.

## Claims

1. A continuous process for growing microorganisms in a circulatory system, comprising supplying a CSL solution to the circulatory system such that some of a mixture of viable bacteria and a culture nutrient liquid is extractible therefrom, adding an alkaline solution to the CSL solution at a point immediately upstream of where the CSL solution is introduced into the circulatory system and/or to the circulatory system so as to neutralize the CSL solution, and detecting the pH values of the neutralized solution and the culture nutrient liquid in the circulatory system so that an adequate amount of the alkaline solution is constantly added.

2. A continuous process as defined in claim 1, wherein the viable bacteria are lactic acid bacteria.

3. A continuous process as defined in claim 1, wherein the detection of the pH value is conducted only on the culture nutrient liquid in the circulatory system.

Fig. 1

Fig. 2

# Fig. 3

# Fig. 4

Fig. 5

Number of Raw Bacteria (y-axis)

Ratio of Extroction to supply (-) (x-axis)

△ : Invention
c : Prior Art

Fig. 6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 698 303  (BAILEY et al.)<br>* Column 5, lines 5-28; column 6, lines 20-66; column 13, lines 5-8; figure 2; column 14, line 1 - column 16, line 40; claims 1-4 *<br>----- | 1-3 | C 12 N   1/00 |

**TECHNICAL FIELDS SEARCHED (Int. Cl.5)**

C 12 N

The present search report has been drawn up for all claims

| Place of search | Date of completion of the search | Examiner |
|---|---|---|
| THE HAGUE | 13-09-1989 | EPAILLARD P.J.H.M. |